# FASCICULE DE BREVET EUROPEEN

(11) **EP 2 456 744 B1**
(45) Date de publication et mention de la délivrance du brevet: **12.12.2018**
(21) Numéro de dépôt: 10729887.9
(22) Date de dépôt: 07.07.2010
(51) Int. Cl.: C07C 51/04, C07C 51/58, C07C 51/60, C07C 51/62, C07C 51/64, C07C 53/48, C07C 53/18

(54) **PROCEDE DE PREPARATION D'UN FLUORURE D'HALOGENOACETYLE ET DE SES DERIVES**
VERFAHREN ZUR HERSTELLUNG EINES HALOGENOACETYLFLUORIDS UND DERIVATEN DARAUS
METHOD FOR PREPARING A HALOGENOACETYL FLUORIDE AND THE DERIVATIVES THEREOF

(30) Priorité: 21.07.2009 FR 0903580
(43) Date de publication de la demande: 30.05.2012
(73) Titulaire: Rhodia Opérations, 75009 Paris (FR)
(72) Inventeur: SAINT-JALMES, Laurent, 69390 Vourles (FR); METZ, François, 69540 Irigny (FR)
(74) Mandataire: Menville, Laure
(86) Numéro de dépôt international: PCT/EP2010/059747
(87) Numéro de publication internationale: WO 2011/009726

(56) Documents cités:
- GB-A- 976 316
- US-A- 5 672 748
- MARKOVSKII, L. N. ET AL: "Applications of dialkylaminosulfur trifluorides for the syntheses of acid fluorides" SYNTHESIS , (12), 801-2 CODEN: SYNTBF; ISSN: 0039-7881, 1975, XP002563224
- DEN W ET AL: "Photooxidation and biotrickling filtration for controlling industrial emissions of trichloroethylene and perchloroethylene" CHEMICAL ENGINEERING SCIENCE, OXFORD, GB, vol. 61, no. 24, 1 décembre 2006 (2006-12-01), pages 7909-7923, XP025011957 ISSN: 0009-2509 [extrait le 2006-12-01]

## Description

La présente invention a pour objet un procédé de préparation d'un fluorure d'halogénoacétyle choisi parmi les chloroacétyles et les bromoacétyles, et de ses dérivés.

L'invention concerne aussi bien la préparation d'un fluorure de monohalogénoacétyle que celle d'un fluorure de polyhalogénoacétyle.

L'invention vise également l'utilisation du fluorure d'halogénoacétyle comme intermédiaires de fabrication du fluorure de fluoroacétyle, de l'acide fluorocarboxylique correspondant et dérivé.

L'invention a trait plus particulièrement à la préparation du fluorure de trichloroacétyle qui est un intermédiaire de fabrication du fluorure de trifluoroacétyle qui conduit à l'acide trifluoroacétique.

Une voie classique de préparation de l'acide trifluoroacétique est d'hydrolyser le fluorure de trifluoroacétyle.

Une méthode de préparation du fluorure de trifluoroacétyle décrite dans la littérature (GB 976 316) est d'effectuer la fluoration du chlorure de trichloroacétyle en faisant réagir ce dernier, avec l'acide fluorhydrique, en présence d'un catalyseur contenant du chrome généralement un oxyde de chrome III.

L'article scientifique de L. N. Markovskii et V. E. Pashinnik (« Applications of Dialkylaminosulfur Trifluorides for the Syntheses of Acid Fluorides ». Synthesis (12), 1975) décrit un méthode de préparation de fluorure de trichloroacétyle à partir de chlorure de trichloroacétyle par réaction avec un trifluorure de sialdylamonium.

Le brevet US 5 672 748 décrit un procédé de préparation de fluorure de trifluoroacétyle par reaction d'un chlorure de trichloroacétyle avec de l'acide fluorhydrique en phase gaz.

Le chlorure de trichloroacétyle est un composé qui est obtenu selon une réaction de photoxydation à savoir, la réaction du tétrachloréthylène (dénommé également perchloréthylène) avec de l'oxygène, en présence de photons. La photoxydation du trichloroethylene et du perchloroéthylène est par example décrite dnas l'article scientifique Den et al (« Photooxidation and biotricklinq filtration for controlling industrial emissions of trichloroethylene and perchloroethylene » Chemical Engineering Science, vol.61, No.24, 2006)

Le problème qui se pose est que cette réaction est une réaction extrêmement lente. La transformation totale du tétrachloroéthylène en chlorure de trichloroacétyle demande des temps de réaction très longs. Par exemple, pour obtenir un taux de transformation quasi-total, un temps de réaction supérieur à une centaine d'heures est nécessaire.

Lorsque l'on souhaite atteindre un taux de transformation élevé, on note une baisse de sélectivité de la réaction suite à la formation de produits secondaires lourds.

Par ailleurs, en fin de réaction de photoxydation du tétrachloréthylène, on obtient un mélange comprenant essentiellement du chlorure de trichloroacétyle et le tétrachloréthylène excédentaire. Or, il apparaît très difficile de séparer lesdits composés car leurs points d'ébullition respectivement de 121°C pour le tétrachloréthylène et de 118°C pour le chlorure de trichloroacétyle sont trop rapprochés pour les séparer par distillation. Ainsi, le produit engagé dans les étapes suivantes n'est pas pur car constitué d'un mélange tel que défini.

Pour pallier ces inconvénients, la Demanderesse propose un procédé qui permet de réduire la durée de la première étape et d'obtenir un intermédiaire réactionnel exempt de son précurseur éthylénique.

Un premier objet de la présente invention est un procédé de préparation d'un fluorure d'halogénoacétyle choisi parmi les chloroacétyles et les bromoacétyles.

Un autre objet de l'invention est un procédé de préparation d'un fluorure de fluoroacétyle faisant intervenir le fluorure d'halogénoacétyle comme produit intermédiaire.

Un autre objet de l'invention est un procédé de préparation d'un acide fluorocarboxylique faisant intervenir le fluorure de fluoroacétyle comme produit intermédiaire.

Il a maintenant été trouvé et c'est ce qui constitue l'objet de la présente invention, un procédé de préparation d'un fluorure d'halogénoacétyle choisi parmi les chloroacétyles et les bramoacétyles caractérisé par le fait qu'il comprend :
- une étape de préparation d'un chlorure ou d'un bromure dudithalogénoacétyle par photoxydation d'un composé éthylénique chloré ou bramé, dans des conditions telles que le taux de transformation du composé éthylénique chloré ou bromé en ledit chlorure ou ledit bromure d'halogénoacétyle est au plus égal à 80 % conduisant à un mélange réactionnel comprenant essentiellement ledit chlorure ou ledit bromure d'halogénoacétyle et le composé éthylénique chloré ou bromé en excès,
- une étape de fluoration partielle du mélange obtenu par réaction de ce dernier avec l'acide fluorhydrique permettant d'obtenir un mélange de fluorure d'halogénoacétyle et du composé éthylénique chloré ou bromé en excès,
- une étape de séparation du fluorure d'halogénoacétyle et du composé éthylénique chloré ou bromé en excès.

Un autre objet de l'invention est un procédé de préparation d'un fluorure de fluoroacétyle à partir du fluorure d'halogénoacétyle précédemment obtenu caractérisé par le fait qu'il comprend en outre :
- une deuxième étape de fluoration du fluorure d'halogénoacétyle obtenu en fluorure de fluoroacétyle par réaction du fluorure d'halogénoacétyle et de l'acide fluorhydrique, en phase gazeuse, en présence d'un catalyseur à base de chrome.

Un autre objet de l'invention est un procédé de préparation d'un acide fluoroacétique à partir du fluorure de fluoroacétyle précédemment obtenu caractérisé par le fait qu'il comprend en outre :
- une étape d'hydrolyse du fluorure de fluoroacétyle obtenu conduisant à un acide fluoroacétique.

On entend dans le présent texte, par « composé halogénoéthylénique », l'éthylène dont un à quatre de ses atomes d'hydrogène peuvent être remplacés par un atome de chlore ou de brome. On peut également le nommer « composé éthylénique chloré ou bromé ».

On désigne par « halogénoacétyle », les chloroacétyles et les bromoacétyles mais plus préférentiellement les chloroacétyles.

On désigne par « halogène », le chlore ou le brome et par « halogénure », un chlorure ou un bromure.

Plus précisément, l'invention a pour objet un procédé de préparation d'un fluorure de chloro- ou bromoacétyle caractérisé par le fait qu'il comprend :
- une étape de préparation d'un chlorure ou bromure de chloro- ou bromoacétyle par photoxydation d'un composé éthylénique chloré ou bromé dans des conditions telles que le taux de transformation du composé éthylénique chloré ou bromé en chlorure ou bromure de chloro- ou bromoacétyle est au plus égal à 80 % conduisant à un mélange réactionnel comprenant essentiellement le chlorure ou le bromure de chloro- ou bromoacétyle et le composé éthylénique chloré ou bromé en excès,
- une étape de fluoration partielle du mélange obtenu par réaction de ce dernier avec l'acide fluorhydrique permettant d'obtenir un mélange de fluorure de chloro- ou bromoacétyle et du composé éthylénique chloré ou bromé en excès,
- une étape de séparation du fluorure de chloro- ou bromoacétyle et du composé éthylénique chloré ou bromé en excès.

L'invention inclut également un procédé de préparation d'un fluorure de fluoroacétyle à partir du fluorure de chloro- ou bromoacétyle précédemment obtenu caractérisé par le fait qu'il comprend en outre :
- une deuxième étape de fluoration du fluorure de chloro- ou bromoacétyle obtenu en fluorure de fluoroacétyle par réaction du fluorure de chloro- ou bromoacétyle et de l'acide fluorhydrique, en phase gazeuse, en présence d'un catalyseur à base de chrome.

Comme mentionné précédemment, l'invention inclut également un procédé de préparation d'un acide fluoroacétique à partir du fluorure de fluoroacétyle précédemment obtenu caractérisé par le fait qu'il comprend en outre une étape d'hydrolyse du fluorure de fluoroacétyle obtenu conduisant à un acide fluoroacétique.

Un mode de réalisation préféré de l'invention consiste en un procédé de préparation de l'acide trifluoroacétique caractérisé par le fait qu'il comprend :
- une étape de préparation du chlorure de trichloroacétyle par photoxydation du tétrachloroéthylène dans des conditions telles que le taux de transformation du tétrachloroéthylène en chlorure de trichloroacétyle est au plus égal à 80 % conduisant à un mélange réactionnel comprenant essentiellement le chlorure de trichloroacétyle et le tétrachloroéthylène en excès,
- une étape de fluoration partielle du mélange obtenu par réaction de ce dernier avec l'acide fluorhydrique permettant d'obtenir un mélange de fluorure de trichloroacétyle et du tétrachloroéthylène en excès,
- une étape de séparation du fluorure de trichloroacétyle du tétrachloroéthylène en excès,
- une deuxième étape de fluoration du fluorure de trichloroacétyle obtenu en fluorure de trifluoroacétyle par réaction du fluorure de trichloroacétyle et de l'acide fluorhydrique, en phase gazeuse, en présence d'un catalyseur à base de chrome,
- une étape d'hydrolyse du fluorure de trifluoroacétyle obtenu.

Selon une caractéristique du procédé de l'invention, on effectue la préparation d'un halogénure d'halogénoacétyle par photoxydation d'un composé halogénoéthylénique.

Le composé halogénoéthylénique intervenant dans le procédé de l'invention répond à la formule suivante : 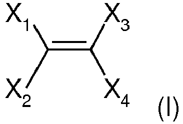 dans ladite formule :
- X₁, X₂, X₃ représentent un atome d'hydrogène ou un atome de chlore ou de brome,
- X₄ représente un atome de chlore ou de brome.

Dans la formule (I), au moins l'un des atomes X₁, X₂, X₃ représente préférentiellement un autre atome de chlore ou de brome.

Comme exemples de composés répondant à la formule (I), on peut citer plus particulièrement le chlorure de vinylidène (1,1-dichloroéthylène), le trichloréthylène et le tétrachloroéthylène.

La réaction est effectuée avantageusement au voisinage de la température d'ébullition du milieu réactionnel généralement entre 50 et 120°C sous irradiation actinique, sous courant d'oxygène pendant une durée qui peut être de 2 à 100 heures.

Il est préférable que l'oxygène soit introduit en excès de préférence à raison de 1 à 10 mol d'oxygène par mole d'halogénoacétyle formé. L'oxygène en excès peut être éventuellement récupéré et recyclé.

La réaction est avantageusement conduite en l'absence d'eau.

Selon une caractéristique du procédé de l'invention, la réaction est poursuivie jusqu'à ce que le taux de transformation du composé halogénoéthylénique en halogénure d'halogénoacétyle soit au plus égal à 80 %, de préférence compris entre 30 et 80 % et encore plus préférentiellement entre 60 et 75 %.

En fin de réaction, on obtient un mélange comprenant essentiellement l'halogénure d'halogénoacétyle et le composé halogénoéthylénique de formule (I) en excès.

L'halogénure d'halogénoacétyle répond à la formule suivante :

X₁X₂X₃C-COX₄ (II)

dans ladite formule :
- X₁, X₂, X₃ et X₄ ont les significations données pour la formule (I).

Le mélange obtenu comprend généralement de 20 à 70 % en masse d'halogénure d'halogénoacétyle et de 70 à 20 % en masse du composé halogénoéthylénique et moins de 10 % d'impuretés comme par exemple le composé saturé halogéné résultant de l'halogénation du composé halogénoéthylénique ou le dimère polyhalogéné du composé halogénoéthylénique.

Selon une deuxième étape du procédé de l'invention, on effectue la préparation de fluorure d'halogénoacétyle en effectuant la fluoration partielle du mélange précédemment obtenu qui contient essentiellement l'halogénure d'halogénoacétyle, par réaction de ce dernier avec de l'acide fluorhydrique liquide.

Bien qu'un catalyseur de fluoration puisse être mis en oeuvre, le mode de réalisation préféré de cette étape de fluoration partielle consiste à conduire la réaction en l'absence de catalyseur.

La quantité d'acide fluorhydrique exprimée par le rapport entre le nombre de moles d'acide fluorhydrique et le nombre de moles d'halogénure d'halogénoacétyle est choisi avantageusement entre 1 et 10. Ledit rapport est compris de préférence entre 1 et 5.

La réaction de fluoration est conduite à une température choisie de préférence entre 80°C et 150°C et plus préférentiellement entre 100°C et 120°C.

La réaction est conduite sous pression autogène des réactifs qui est régulée entre 1 et 100 bar (absolu), et de préférence entre 5 et 30 bar de manière à éliminer l'acide halohydrique (généralement l'acide chlorhydrique) formé au cours de la réaction.

On met en oeuvre la réaction sous atmosphère de gaz inertes, de préférence sous atmosphère d'azote.

En fin de réaction, on obtient un milieu biphasique.

L'une des phases est constituée par l'acide fluorhydrique en excès et l'autre phase est une phase organique qui comprend le fluorure d'halogénoacétyle et le composé halogénoéthylénique qui n'a pas réagi.

On sépare les deux phases selon les techniques classiques de séparation comme par exemple la décantation.

La phase supérieure récupérée comprend de l'acide fluorhydrique qui peut être recyclé aux étapes de fluoration.

La phase inférieure est une phase organique.

La composition de la phase organique peut varier mais comprend de préférence de 50 à 80 % en masse de fluorure d'halogénoacétyle et de 50 à 20 % en masse de composé halogénoéthylénique.

La composition préférée de la phase organique est de 60 à 70 % en masse de fluorure d'halogénoacétyle et de 40 à 30 % en masse de composé halogénoéthylénique.

Dans une étape suivante, on procède à une opération de distillation ce qui permet d'effectuer la séparation du fluorure d'halogénoacétyle et du composé halogénoéthylénique.

On introduit la phase organique précitée comprenant les composés à séparer dans une colonne de distillation, et où l'on élimine en tête de distillation, de l'acide fluorhydrique puis le composé ayant le point d'ébullition le moins élevé entre le fluorure d'halogénoacétyle et le composé halogénoéthylénique et l'on récupère en pied de distillation le composé ayant le plus haut point d'ébullition.

La distillation est conduite à une température dans le bouilleur généralement supérieure de 20°C à 50°C à la température d'ébullition du composé éliminé en tête de distillation et sous une pression comprise entre 400 mbar et 10 bar, de préférence aux environs de 1 bar.

La distillation est effectuée dans un appareil de distillation classique.

L'homme du métier est parfaitement en mesure de choisir les moyens à mettre en oeuvre en fonction des composés à séparer.

On rappellera simplement ce qui suit. La taille (notamment le diamètre) des colonnes de distillation dépend du flux circulant et de la pression interne. Leur dimensionnement se fera donc principalement suivant le débit de mélange à traiter. Le paramètre interne qu'est le nombre d'étages théoriques est déterminé notamment par la pureté du composé de départ et la pureté du produit devant être obtenus en tête de distillation.

On précisera que les colonnes pourront être garnies indifféremment de plateaux ou de garnissage ordonné, comme cela est parfaitement connu de l'homme du métier.

L'installation étant déterminée, l'homme du métier ajuste les paramètres de fonctionnement de la colonne.

Ainsi, la colonne de distillation pourra être avantageusement, mais non limitativement, une colonne ayant les spécifications suivantes :
- nombre d'étages théoriques : de 1 à 10, de préférence de 1 à 5,
- taux de reflux R compris entre 1 et 50, de préférence entre 10 et 20.

En bas de colonne, on récupère un culot de distillation comprenant selon les cas, le fluorure d'halogénoacétyle ou le composé halogénoéthylénique et en tête de colonne une phase gazeuse constituée par l'acide fluorhydrique puis par le fluorure d'halogénoacétyle ou le composé halogénoéthylénique.

On refroidit la phase gazeuse et la transforme sous forme liquide par refroidissement à une température par exemple comprise entre -20°C et 10°C, de préférence comprise entre -10°C et 0°C.

Cette opération est conduite par passage dans un condenseur qui est un appareil classique par exemple un échangeur tubulaire alimenté par de l'eau ou par un fluide maintenu à une température voisine de la température de refroidissement choisie.

On obtient donc en pied de distillation soit le composé halogénoéthylénique, soit le fluorure d'halogénoacétyle.

On recycle le composé halogénoéthylénique et l'on traite ensuite le fluorure d'halogénoacétyle obtenu.

Dans le cas de la séparation du fluorure de trichloroacétyle du tétrachloroéthylène, on récupère en tête de distillation l'acide fluorhydrique puis le fluorure de trichloroacétyle et en pied de distillation, le tétrachloroéthylène qui peut être recyclé à la première étape du procédé.

Selon une caractéristique du procédé de l'invention, on effectue la préparation de fluorure de fluoroacétyle par réaction du fluorure d'halogénoacétyle et de l'acide fluorhydrique, en phase gazeuse, en présence d'un catalyseur à base de chrome.

Intervient dans le procédé de l'invention, un catalyseur de fluoration à base de chrome.

Le catalyseur utilisé comprend de préférence les oxydes, halogénures, oxyhalogénures ou sels minéraux de chrome éventuellement dopé par un élément métallique tel que par exemple le nickel, le cobalt, le magnésium et le zinc.

Il s'agit préférentiellement d'un oxyde de chrome, d'un fluorure de chrome ou d'un oxyfluorure de chrome ou bien du chrome dopé par un élément métallique comme par exemple, le nickel et le magnésium.

Le catalyseur peut subir une activation par un traitement thermique et/ou un traitement de fluoration. En particulier, l'activation peut avoir lieu lors de la fluoration. La température est avantageusement choisie entre 100°C et 400°C, de préférence entre 200°C et 300°C.

On utilise en particulier le chrome sous forme d'oxydes sous différents degrés d'oxydation et/ou sous forme d'hydroxydes sous forme de poudre ou de gel.

On peut mettre en oeuvre un oxyde de chrome III activé préparé par exemple par précipitation de sels de chrome III hydrosolubles comme par exemple, les chlorures, nitrates, acétates, sulfates à l'aide d'un hydroxyde de métal alcalin, de préférence le sodium ou le potassium ou d'ammonium. Le précipité est séché à environ 110°C et calciné à une température inférieure à 700°C, de préférence comprise entre 400 et 600°C.

L'oxyde de chrome anhydre peut être obtenu par calcination de sels de chrome inorganiques comme le chromate d'ammonium ou le nitrate de chrome ou par calcination de sels de chrome organiques comme par exemple, les oxalates ou formiates de chrome à 350°C, sous atmosphère d'azote.

On peut faire appel notamment à un catalyseur de type Cr-Ni, avec une valence du chrome comprise entre 2 et 3 et celle du nickel comprise entre 0 et 2, la quantité de nickel exprimée en % atomique représentant de 0,1 à 10 %.

Un mode de préparation de ce catalyseur consiste à faire une décomposition thermique, séparément ou en mélange d'un ou plusieurs sels organiques du chrome (par exemple oxalate) et d'un sel ou plusieurs sels du nickel (par exemple oxalate), mise en forme du mélange puis fluoration du catalyseur mis en forme.

La décomposition thermique a lieu généralement entre 370°C et 400°C, sous atmosphère de gaz inerte, par exemple l'azote.

La mise en forme du catalyseur obtenu peut être faite, dans des conditions non oxydantes, par exemple par extrusion puis le produit mis en forme est séché vers 120°C - 130°C, puis calciné à 370°C - 400°C, sous atmosphère inerte.

Le catalyseur est chauffé entre 100°C et 500°C, sous acide fluorhydrique entre 1 et 12 heures.

Un catalyseur de type Cr-Mg peut également être mis en oeuvre.

Il peut être obtenu notamment par mélange d'un sel de chrome (par exemple nitrate) en solution avec un oxyde ou hydroxyde de magnésium, séchage prolongée entre 12 et 24 heures par exemple à 100°C, puis activation par l'acide fluorhydrique, par exemple à 200°C.

La phase active peut être apportée sous une forme finement divisée ou bien mise en forme ou déposé sur un support.

Comme exemples de supports, on peut mentionner la silice, l'alumine, la zircone, l'oxyde de titane. De préférence, le chrome est déposé sur un support à raison de 0,5 % à 5 % de la masse du catalyseur.

Les catalyseurs peuvent se présenter sous différentes formes dans le procédé de l'invention : poudre, produits mis en forme tels que granulés (par exemple, extrudés ou billes), pastilles, qui sont obtenus par extrusion, moulage, compactage ou tout autre type de procédé connu. En pratique, sur le plan industriel, ce sont les formes de granulés ou de billes qui présentent le plus d'avantages tant sur le plan de l'efficacité que sur le plan de commodité de mise en oeuvre.

Conformément au procédé de l'invention, on effectue la réaction de fluoration en faisant réagir le fluorure d'halogénoacétyle et l'acide fluorhydrique, en phase gazeuse, en présence du catalyseur de fluoration.

Le rapport entre l'acide fluorhydrique et le fluorure d'halogénoacétyle peut varier largement. Généralement, la quantité de l'acide fluorhydrique est excédentaire. Ainsi, le rapport entre le nombre de moles d'acide fluorhydrique et le nombre de moles de fluorure d'halogénoacétyle varie le plus souvent entre 1 et 20. Il est avantageusement choisi entre 6 et 8.

On réalise le procédé conforme à l'invention à une température élevée, en règle générale supérieure à 200°C. Il est recommandé de travailler à des températures comprises entre 250°C et 400°C, de préférence comprise entre 250°C et 300°C.

Pour des raisons de simplicité, on réalise le procédé de l'invention sous la pression atmosphérique. Toutefois, il est également possible d'opérer sous des pressions plus basses ou plus élevées.

D'un point de vue pratique, le procédé peut être mis en oeuvre en discontinu ou en continu.

On commence par effectuer le mélange d'une façon quelconque, du fluorure d'halogénoacétyle et de l'acide fluorhydrique.

Ainsi, on peut mélanger lesdits réactifs, dans une zone de mélange, puis envoyer le mélange obtenu sur le lit catalytique.

Lorsque l'on réalise le procédé en discontinu, la quantité de catalyseur de fluoration mis en oeuvre, exprimée en masse de catalyseur par masse du fluorure d'halogénoacétyle peut varier, par exemple, entre 0,5 et 20 %, de préférence entre 0,5 et 5 %.

L'autre variante de l'invention consiste à conduire la réaction en continu, dans un réacteur tubulaire comportant le catalyseur solide disposé en lit fixe.

Le fluorure d'halogénoacétyle et l'acide fluorhydrique peuvent être introduits séparément ou en mélange dans le réacteur. Comme mentionné précédemment, on peut les mélanger dans une zone de mélange, puis envoyer le mélange obtenu sur le lit catalytique.

Le mélange réactionnel traverse le lit catalytique, de préférence, de bas en haut.

Le temps de contact qui est défini comme le rapport entre le volume apparent de catalyseur et le débit du flux gazeux peut varier largement, et est le plus souvent compris entre 0,2 et 100 secondes. Le temps de contact est choisi de préférence entre 5 et 50 secondes.

La masse de substrat mise en oeuvre par masse de catalyseur et par heure varie généralement entre 0,01 h⁻¹ et 2 h⁻¹, de préférence entre 0,05 h⁻¹ et 0,5 h⁻¹.

En fin de réaction, on récupère une phase gazeuse comprenant le fluorure de fluoroacétyle, l'acide fluorhydrique en excès, l'acide halohydrique (de préférence l'acide chlorhydrique) formé par la réaction.

Selon une variante de l'invention, on peut récupérer le fluorure de fluoroacétyle à partir dudit flux gazeux comprenant le fluorure de fluoroacétyle, les acides fluorhydrique et halohydrique en condensant ledit flux gazeux par abaissement de sa température entre - 40°C à 10°C, de préférence entre -20°C à 0°C puis en effectuant la distillation du flux condensé.

Conformément au procédé de l'invention, on effectue ensuite une étape d'hydrolyse du fluorure de fluoroacétyle en acide fluorocarboxylique.

A cet effet, on met le flux gazeux en présence d'eau. La quantité d'eau mise en oeuvre est au moins égale à la quantité soetchiométrique.

Généralement, l'opération est effectuée en envoyant le flux gazeux dans une colonne d'hydrolyse : l'eau étant envoyée à contre-courant du flux gazeux qui remonte de bas en haut dans la colonne.

On peut également effectuer une hydrolyse acide, par exemple en faisant appel à une solution d'un acide minéral fort, par exemple de l'acide chlorhydrique à 30 % en masse.

Ainsi, on récupère en pied de colonne, l'acide fluorocarboxylique et en tête de colonne, l'acide chlorhydrique gazeux.

Les sels des acides fluorocarboxyliques peuvent être aisément fabriqués à partir de l'acide, notamment par réaction avec une base, de préférence la soude ou la potasse.

Le procédé de l'invention est avantageusement conduit dans un appareillage susceptible de résister à la corrosion provoquée par l'acide fluorhydrique. A cet effet, on choisit des matériaux pour la partie en contact avec le milieu réactionnel résistant à la corrosion comme les alliages à base de molybdène, chrome, cobalt, fer, cuivre manganèse, titane, zirconium, aluminium, carbone et tungstène vendus sous les marques HASTELLOY® ou les alliages de nickel, chrome, fer, manganèse additivés de cuivre et/ou molybdène commercialisés sous la dénomination INCONEL® et plus particulièrement les alliages HASTELLOY C 276 ou INCONEL 600, 625 ou 718.

On peut choisir également les aciers inoxydables, tels que les aciers austénitiques [Robert H. Perry et al, Perry's Chemical Engineers' Handbook, Sixth Edition (1984), page 23-44]. et plus particulièrement les aciers inoxydables 304, 304 L, 316 ou 316 L. On met en oeuvre un acier ayant une teneur en nickel au plus de 22 % en masse, de préférence comprise entre 6 et 20 %, et plus préférentiellement comprise entre 8 et 14 %.

Les aciers 304 et 304 L ont une teneur en nickel variant entre 8 et 12 % et les aciers 316 et 316 L ont une teneur en nickel variant entre 10 et 14 %.

L'ensemble des différentes étapes du procédé de l'invention peuvent être mises en oeuvre en continu ou en discontinu.

Le procédé de l'invention est particulièrement intéressant car il présente de nombreux avantages.

La première étape du procédé qui requiert une limitation du taux de transformation du composé halogénoéthylénique, permet d'obtenir un gain sur la durée de la réaction et sur la sélectivité de la réaction.

De plus, en raison d'un temps de réaction plus court, on évite la fluoration de l'halogénoéthylénique. En effet, par exemple le tétrachloroéthylène se fluore et forme un composé chlorofluorocarboné CFC considéré comme un gaz à effet de serre.

Le procédé de l'invention permet à la fin de la première étape de fluoration de séparer le fluorure d'halogénoacétyle obtenu du composé halogénoéthylénique en excès ce qui permet d'engager un produit plus pur dans la suite du procédé.

Par ailleurs, la deuxième étape de fluoration requiert moins d'acide fluorhydrique.

Les exemples qui suivent, illustrent l'invention sans toutefois la limiter. Les abréviations ont les significations suivantes :
- CTCA : chlorure de trichloroacétyle
- FTCA : fluorure de trichloroacétyle
- FTFA : fluorure de trifluoroacétyle
- TFA : acide trifluoroacétique
- PER : perchloréthylène (ou tétrachloroéthylène)

Dans les exemples, on définit le taux de conversion et les rendements obtenus.

Le taux de conversion (TT) correspond au rapport entre le nombre de moles de substrat CTCA transformées et le nombre de moles de substrat CTCA engagées.

Le rendement (RT) correspond au rapport entre le nombre de moles de produit FTCA formées et le nombre de moles de substrat CTCA transformées.

### Exemple 1

Dans un réacteur en Pyrex de 2 litres, à double enveloppe et équipé d'un condenseur (-10°C) et d'une lampe à vapeur de mercure, on introduit 500 g de PER que l'on porte à reflux (110°C) sous irradiation (210-260 nm) et sous courant gazeux, constitué d'un mélange d'oxygène pur (4 L/h) et d'azote (0,5 L/h).

L'analyse par chromatographie en phase gazeuse (détection catharométrique) du milieu réactionnel après 4 h de réaction indique la présence de 74 % de CTCA, de 26 % de PER (pourcentages massiques).

Dans un autoclave de 0,3 litre en Hastelloy® C276, refroidi à 0°C, on introduit 210,6 g (0,86 mol) d'un mélange CTCA/PER (74/26 p/p) : et 40 g (2 mol) d'acide fluorhydrique anhydre.

Le réacteur est ensuite chauffé à 120°C (montée en température d'environ 1 h) puis maintenu à 120°C pendant 5 h sous pression autogène (la pression étant d'environ 60 bar).

Le réacteur est ensuite refroidi à 0°C (pression résiduelle d'environ 15 bar), puis le milieu réactionnel est lentement soutiré dans un flacon en polyfluoroéthylène refroidi à - 30°C.

Le milieu réactionnel biphasique est alors dégazé à 20°C sous agitation pour éliminer HCI et HF présents.

Après dégazage, le milieu réactionnel, homogène, est analysé par chromatographie en phase gazeuse quantitative pour conduire aux résultats suivants :
- TT_{CTCA} : 89 %
- RT_{FTCA} : 92 %

Le milieu réactionnel brut final obtenu est constituée de deux phases dont les compositions sont les suivantes :
- phase supérieure :
   - HF = 94 % en mol
   - FTCA = 1,5 % en masse
   - PER = 0,07 % en masse (soit 0,25 % du PER initial)
   - CTCA = HCl = 0 %
- phase inférieure :
   - HF = 1 % en masse
   - FTCA = 98,5 % en mol
   - PER = 93 % du PER initial
   - CTCA = 7 % du CTCA initial
   - HCl = 1,1 % en masse

On sépare les phases aqueuse et organique, après décantation.

On récupère le FTCA à partir de la phase organique inférieure par distillation sous pression atmosphérique.

On récupère en tête de distillation, le mélange des acides halohydriques puis le FTCA qui distille à 69°C.

Le PER se retrouve en pied de distillation avec le CTCA non réagi.

On effectue la fluoration du FTCA.

Dans un réacteur en Hastelloy C276 constitué d'un tube de 60 cm de longueur et de diamètre extérieur de 2,5 cm, rempli d'un catalyseur à base d'oxyde de chrome (∼150 g) préalablement séché jusqu'à poids constant et fluoré (24 heures à 250°C), on introduit du fluorure de trichloroacétyle (FTCA) préalablement distillé à un débit de 20 g/h et de l'HF anhydre à un débit de 20 à 50 g/h.

La température est fixée à 250-300°C.

Dans ces conditions, le temps de séjour tₛ varie entre 10 et 20 s.

On obtient le fluorure de trifluoroacétyle avec un rendement pondéral de 90 %.

Le fluorure de trifluoroacétyle est hydrolysé en acide trifluoroacétique à l'aide d'eau acidifiée par HCl.

Le rendement de la réaction d'hydrolyse est supérieure à 90 % en masse.

### Exemples 2 à 4

Les exemples suivants concernant la fluoration du CTCA en FTCA ont été menés dans les mêmes conditions générales (température = 120°C) ; seuls les paramètres variant sont mentionnés dans le tableau (I) ci-dessous.

Dans les exemples 3 et 4, la réaction est conduite sous pression autogène alors que dans l'exemple 4, la pression est régulée à 22 bar.

Les résultats obtenus sont consignés dans le tableau suivant :

**Tableau (I)**

| Ex | CTCA/PER (g) | HF/CTCA éq. | t (h) | Pression finale à 120°C (bar) | TT_{CTCA} % | TT_{PER} % | RT_{FTCA} % |
|---|---|---|---|---|---|---|---|
| 2 | 211 | 2,3 | 6 | 35 | 86 | 8,5 | 87 |
| 3 | 244 | 1,3 | 6 | 63 | 81 | 7 | 94 |
| 4 | 217 | 2,3 | 5 | régulée 22 bar | 80 | 15 | 90 |

## Revendications

1. Procédé de préparation d'un fluorure d'halogénoacétyle où l'halogénoacétyle est choisi parmi les chloroacétyles et les bromoacétyles **caractérisé par le fait qu'**il comprend :
- une étape de préparation d'un chlorure ou d'un bromure dudit halogénoacétyle par photoxydation d'un composé éthylénique chloré ou bromé, ledit composé éthylénique chloré ou bromé répondant à la formule suivante : 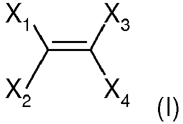 dans ladite formule :
- X₁, X₂, X₃ représentent un atome d'hydrogène ou un atome de chlore ou de brome,
- X₄ représente un atome de chlore ou de brome,
dans des conditions telles que le taux de transformation dudit composé éthylénique en ledit chlorure ou ledit bromure d'halogénoacétyle est au plus égal à 80 % conduisant à un mélange réactionnel comprenant essentiellement ledit chlorure ou ledit bromure d'halogénoacétyle et ledit composé éthylénique en excès,
- une étape de fluoration partielle du mélange obtenu par réaction de ce dernier avec l'acide fluorhydrique permettant d'obtenir un mélange de fluorure dudit halogénoacétyle et dudit composé éthylénique en excès,
- une étape de séparation dudit fluorure d'halogénoacétyle et dudit composé éthylénique en excès.

2. Procédé de préparation d'un fluorure de fluoroacétyle **caractérisé par le fait qu'**il comprend :
- une étape de préparation d'un fluorure d'halogénoacétyle selon le procédé de la revendication 1 ;
- une deuxième étape de fluoration dudit fluorure d'halogénoacétyle obtenu en fluorure de fluoroacétyle par réaction dudit fluorure d'halogénoacétyle et de l'acide fluorhydrique, en phase gazeuse, en présence d'un catalyseur à base de chrome.

3. Procédé de préparation d'un acide fluoroacétique **caractérisé par le fait qu'**il comprend :
- une étape de préparation d'un fluorure de fluoroacétyle selon le procédé de la revendication 2 ;
- une étape d'hydrolyse du fluorure de fluoroacétyle obtenu conduisant à un acide fluoroacétique.

4. Procédé selon l'une des revendications 1 à 3 **caractérisé par le fait que** ledit composé éthylénique est le chlorure de vinylidène, le trichloréthylène ou le tétrachloroéthylène.

5. Procédé selon l'une des revendications 1 à 4 **caractérisé par le fait que** le taux de transformation dudit composé éthylénique en ledit chlorure ou ledit bromure d'halogénoacétyle est compris entre 30 et 80 % et de préférence entre 60 et 75 %.

6. Procédé selon l'une des revendications 1 à 5 **caractérisé par le fait que** le mélange obtenu comprend de 20 à 70 % en masse dudit chlorure ou dudit bromure d'halogénoacétyle et de 70 à 20 % en masse dudit composé éthylénique et moins de 10 % d'impuretés.

7. Procédé selon l'une des revendications 1 à 6 **caractérisé par le fait que** l'on effectue la fluoration partielle du mélange obtenu par réaction de ce dernier avec de l'acide fluorhydrique liquide : le rapport entre le nombre de moles d'acide fluorhydrique et le nombre de moles dudit chlorure ou dudit bromure d'halogénoacétyle étant choisi entre 1 et 10 et de préférence entre 1 et 5.

8. Procédé selon la revendication 7 **caractérisé par le fait que** la réaction de fluoration est conduite à une température choisie entre 80°C et 150°C et de préférence entre 100°C et 120°C, sous pression autogène des réactifs.

9. Procédé selon l'une des revendications 7 et 8 **caractérisé par le fait que** l'on obtient en fin de réaction un mélange biphasique dont la phase organique comprend de 50 à 80 % en masse, de préférence de 60 à 70 % dudit fluorure d'halogénoacétyle et de 50 à 20 % en masse, de préférence de 40 à 30 % dudit composé éthylénique.

10. Procédé selon l'une des revendications 1 à 9 **caractérisé par le fait que** l'on procède à une opération de distillation ce qui permet d'effectuer la séparation dudit fluorure d'halogénoacétyle et dudit composé éthylénique et que l'on recycle éventuellement ledit composé éthylénique.

11. Procédé selon l'une des revendications 2 à 10 **caractérisé par le fait que** l'on effectue la fluoration dudit fluorure d'halogénoacétyle par réaction de ce dernier avec l'acide fluorhydrique, en présence d'un catalyseur de fluoration : le rapport entre le nombre de moles d'acide fluorhydrique et le nombre de moles dudit fluorure d'halogénoacétyle varie entre 1 et 20, de préférence entre 6 et 8.

12. Procédé selon la revendication 11 **caractérisé par le fait que** le catalyseur de fluoration comprend les oxydes, halogénures, oxyhalogénures ou sels minéraux de chrome éventuellement dopé par un élément métallique de préférence le nickel, le cobalt, le magnésium et le zinc.

13. Procédé selon la revendication 12 **caractérisé par le fait que** le catalyseur de fluoration comprend un oxyde de chrome, un fluorure de chrome ou un oxyfluorure de chrome ou bien du chrome dopé par un élément métallique, de préférence le nickel ou le magnésium.

14. Procédé selon l'une des revendications 11 à 13 **caractérisé par le fait que** le catalyseur subit une activation par un traitement thermique et/ou un traitement de fluoration : l'activation pouvant avoir lieu lors de la fluoration.

15. Procédé selon l'une des revendications 11 à 14 **caractérisé par le fait que** le catalyseur est un oxyde de chrome III.

16. Procédé selon la revendication 11 **caractérisé par le fait que** la température de la réaction est comprise entre 250°C et 400°C, de préférence entre 250°C et 300°C.

17. Procédé selon l'une des revendications 11 à 16 **caractérisé par le fait que** l'on effectue le mélange dudit fluorure d'halogénoacétyle et de l'acide fluorhydrique dans une zone de mélange, puis l'on envoie le mélange obtenu sur le lit catalytique.

18. Procédé selon l'une des revendications 11 à 17 **caractérisé par le fait que** le temps de contact défini comme le rapport entre le volume apparent de catalyseur et le débit du flux gazeux varie entre 0,2 et 100 secondes, de préférence entre 5 et 50 secondes.

19. Procédé selon l'une des revendications 11 à 18 **caractérisé par le fait que** le poids de substrat mis en oeuvre par poids de catalyseur et par heure varie entre 0,01 h⁻¹ et 2 h⁻¹, de préférence entre 0,05 h⁻¹ et 0,5 h⁻¹.

20. Procédé selon l'une des revendications 11 à 19 **caractérisé par le fait que** l'on récupère en fin de réaction, une phase gazeuse comprenant le fluorure de fluoroacétyle, l'acide fluorhydrique en excès, l'acide halohydrique formé par la réaction.

21. Procédé selon l'une des revendications 3 à 20 **caractérisé par le fait que** l'on effectue ensuite une étape d'hydrolyse du fluorure de fluoroacétyle en acide fluoroacétique par mise en présence du flux gazeux avec de l'eau.

22. Procédé selon la revendication 20 **caractérisé par le fait que** l'on récupère le fluorure de fluoroacétyle à partir dudit flux gazeux comprenant le fluorure de fluoroacétyle, les acides fluorhydrique et halohydrique en condensant ledit flux gazeux par abaissement de sa température entre -40°C à 10°C, de préférence entre -20°C à 0°C puis en effectuant la distillation du flux condensé.

23. Procédé de préparation de l'acide trifluoroacétique selon l'une des revendications 1 à 22 **caractérisé par le fait qu'**il comprend :
- une étape de préparation du chlorure de trichloroacétyle par photoxydation du tétrachloroéthylène dans des conditions telles que le taux de transformation du tétrachloroéthylène en chlorure de trichloroacétyle est au plus égal à 80 % conduisant à un mélange réactionnel comprenant essentiellement le chlorure de trichloroacétyle et le tétrachloroéthylène en excès,
- une étape de fluoration partielle du mélange obtenu par réaction de ce dernier avec l'acide fluorhydrique permettant d'obtenir un mélange de fluorure de trichloroacétyle et du tétrachloroéthylène en excès,
- une étape de séparation du fluorure de trichloroacétyle du tétrachloroéthylène en excès,
- une deuxième étape de fluoration du fluorure de trichloroacétyle obtenu en fluorure de trifluoroacétyle par réaction du fluorure de trichloroacétyle et de l'acide fluorhydrique, en phase gazeuse, en présence d'un catalyseur à base de chrome,
- une étape d'hydrolyse du fluorure de trifluoroacétyle obtenu.

24. Procédé selon l'une des revendications 21 et 23 **caractérisé par le fait que** l'on fait réagir l'acide fluoroacétique obtenu avec une base, de préférence la soude ou la potasse.

## Patentansprüche

1. Verfahren zur Herstellung eines Halogenacetylfluorids, wobei das Halogenacetyl aus Chloracetylen und Bromacetylen ausgewählt ist, **dadurch gekennzeichnet, dass** es Folgendes umfasst:
- einen Schritt der Herstellung eines Chlorids oder eines Bromids des Halogenacetyls durch Photooxidation einer Chlor- oder Bromethylenverbindung, wobei die Chlor- oder Bromethylenverbindung der folgenden Formel entspricht: 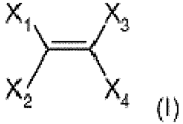 in der:
- X₁, X₂ und X₃ für ein Wasserstoffatom oder ein Chlor- oder Bromatom stehen,
- X₄ für ein Chlor- oder Bromatom steht,
unter solchen Bedingungen, dass der Grad der Umwandlung der Ethylenverbindung in das Halogenacetylchlorid oder -bromid höchstens gleich 80 % ist, was eine Reaktionsmischung ergibt, die im Wesentlichen das Halogenacetylchlorid oder -bromid und die überschüssige Ethylenverbindung umfasst,
- einen Schritt der Teilfluorierung der erhaltenen Mischung durch Umsetzung der Mischung mit Fluorwasserstoffsäure, was den Erhalt einer Mischung des Halogenacetylfluorids und der überschüssigen Ethylenverbindung ermöglicht,
- einen Schritt der Trennung des Halogenacetylfluorids und der überschüssigen Ethylenverbindung.

2. Verfahren zur Herstellung eines Fluoracetylfluorids, **dadurch gekennzeichnet, dass** es Folgendes umfasst:
- einen Schritt der Herstellung eines Halogenacetylfluorids nach dem Verfahren von Anspruch 1;
- einen zweiten Schritt der Fluorierung des erhaltenen Halogenacetylfluorids zu Fluoracetylfluorid durch Umsetzung des Halogenacetylfluorids mit Fluorwasserstoff in der Gasphase in Gegenwart eines Katalysators auf Basis von Chrom.

3. Verfahren zur Herstellung einer Fluoressigsäure, **dadurch gekennzeichnet, dass** es Folgendes umfasst:
- einen Schritt der Herstellung eines Fluoracetylfluorids nach dem Verfahren von Anspruch 2;
- einen Schritt der Hydrolyse des erhaltenen Fluoracetylfluorids, was eine Fluoressigsäure ergibt.

4. Verfahren nach einem der Ansprüche 1 bis 3, **dadurch gekennzeichnet, dass** es sich bei der Ethylenverbindung um Vinylidenchlorid, Trichlorethylen oder Tetrachlorethylen handelt.

5. Verfahren nach einem der Ansprüche 1 bis 4, **dadurch gekennzeichnet, dass** der Grad der Umwandlung der Ethylenverbindung in das Halogenacetylchlorid oder -bromid zwischen 30 und 80 % und vorzugsweise zwischen 60 und 75 % liegt.

6. Verfahren nach einem der Ansprüche 1 bis 5, **dadurch gekennzeichnet, dass** die erhaltene Mischung 20 bis 70 Massen-% des Halogenacetylchlorids oder -bromids und 70 bis 20 Massen-% der Ethylenverbindung und weniger als 10 % Verunreinigungen umfasst.

7. Verfahren nach einem der Ansprüche 1 bis 6, **dadurch gekennzeichnet, dass** die Teilfluorierung der erhaltenen Mischung durch Umsetzung der Mischung mit flüssiger Fluorwasserstoffsäure durchgeführt wird, wobei das Verhältnis zwischen der Zahl von Molen Fluorwasserstoffsäure und der Zahl von Molen des Halogenacetylchlorids oder -bromids zwischen 1 und 10 und vorzugsweise zwischen 1 und 5 liegt.

8. Verfahren nach Anspruch 7, **dadurch gekennzeichnet, dass** die Fluorierungsreaktion bei einer Temperatur zwischen 80 °C und 150 °C und vorzugsweise zwischen 100 °C und 120 °C unter autogenem Druck der Reaktanten durchgeführt wird.

9. Verfahren nach einem der Ansprüche 7 und 8, **dadurch gekennzeichnet, dass** man am Ende der Umsetzung eine zweiphasige Mischung erhält, deren organische Phase 50 bis 80 Massen-%, vorzugsweise 60 bis 70 %, des Halogenacetylfluorids und 50 bis 20 Massen-%, vorzugsweise 40 bis 30 %, der Ethylenverbindung umfasst.

10. Verfahren nach einem der Ansprüche 1 bis 9, **dadurch gekennzeichnet, dass** ein Destillationsarbeitsgang durchgeführt wird, der es ermöglicht, die Trennung des Halogenacetylfluorids und der Ethylenverbindung durchzuführen, und dass die Ethylenverbindung gegebenenfalls rezykliert wird.

11. Verfahren nach einem der Ansprüche 2 bis 10, **dadurch gekennzeichnet, dass** die Fluorierung des Halogenacetylfluorids durch Umsetzung des letzteren mit Fluorwasserstoffsäure in Gegenwart eines Fluorierungskatalysators durchgeführt wird, wobei das Verhältnis zwischen der Zahl von Molen Fluorwasserstoffsäure und der Zahl von Molen des Halogenacetylfluorids zwischen 1 und 20, vorzugsweise zwischen 6 und 8, variiert.

12. Verfahren nach Anspruch 11, **dadurch gekennzeichnet, dass** der Fluorierungskatalysator Oxide, Halogenide, Oxidhalogenide oder anorganische Salze von Chrom, das gegebenenfalls mit einem Metallelement, vorzugsweise Nickel, Cobalt, Magnesium und Zink, dotiert ist, umfasst.

13. Verfahren nach Anspruch 12, **dadurch gekennzeichnet, dass** der Fluorierungskatalysator ein Oxid von Chrom, ein Fluorid von Chrom oder ein Oxidfluorid von Chrom oder auch Chrom, das mit einem Metallelement, vorzugsweise Nickel oder Magnesium, dotiert ist, umfasst.

14. Verfahren nach einem der Ansprüche 11 bis 13, **dadurch gekennzeichnet, dass** der Katalysator einer Aktivierung durch eine Wärmebehandlung und/oder eine Fluorierungsbehandlung unterworfen wird, wobei die Aktivierung während der Fluorierung stattfinden kann.

15. Verfahren nach einem der Ansprüche 11 bis 14, **dadurch gekennzeichnet, dass** es sich bei dem Katalysator um Chrom(III)-oxid handelt.

16. Verfahren nach Anspruch 11, **dadurch gekennzeichnet, dass** die Temperatur der Umsetzung zwischen 250 °C und 400 °C, vorzugsweise zwischen 250 °C und 300 °C, liegt.

17. Verfahren nach einem der Ansprüche 11 bis 16, **dadurch gekennzeichnet, dass** das Mischen des Halogenacetylfluorids und der Fluorwasserstoffsäure in einer Mischzone durchgeführt wird und die erhaltene Mischung dann der Katalysatorschüttung zugeführt wird.

18. Verfahren nach einem der Ansprüche 11 bis 17, **dadurch gekennzeichnet, dass** die Kontaktzeit, die als das Verhältnis zwischen dem scheinbaren Katalysatorvolumen und der Durchflussrate des Gasstroms definiert ist, zwischen 0,2 und 100 Sekunden, vorzugsweise zwischen 55 Sekunden, liegt.

19. Verfahren nach einem der Ansprüche 11 bis 18, **dadurch gekennzeichnet, dass** das eingesetzte Substratgewicht pro Katalysatorgewicht und pro Stunde zwischen 0,01 h⁻¹ und 2 h⁻¹, vorzugsweise zwischen 0,05 h⁻¹ und 0,5 h⁻¹, variiert.

20. Verfahren nach einem der Ansprüche 11 bis 19, **dadurch gekennzeichnet, dass** man am Ende der Umsetzung eine Gasphase, die das Fluoracetylfluorid, die überschüssige Fluorwasserstoffsäure und die bei der Umsetzung angefallene Halogenwasserstoffsäure umfasst, gewinnt.

21. Verfahren nach einem der Ansprüche 3 bis 20, **dadurch gekennzeichnet, dass** anschließend ein Schritt der Hydrolyse des Fluoracetylfluorids zu Fluorwasserstoffsäure durch Inberührungbringen des Gasstroms mit Wasser durchgeführt wird.

22. Verfahren nach Anspruch 20, **dadurch gekennzeichnet, dass** das Fluoracetylfluorid aus dem Gasstrom, der das Fluoracetylfluorid, die Fluorwasserstoffsäure und die Halogenwasserstoffsäure umfasst, durch Kondensieren des Gasstroms durch Senkung seiner Temperatur auf zwischen -40 °C und 10 °C, vorzugsweise zwischen -20 °C und 0 °C, und nachfolgende Destillation des kondensierten Stroms gewonnen wird.

23. Verfahren zur Herstellung von Trifluoressigsäure nach einem der Ansprüche 1 bis 22, **dadurch gekennzeichnet, dass** es Folgendes umfasst:
- einen Schritt der Herstellung von Trichloracetylchlorid durch Photooxidation von Tetrachlorethylen unter solchen Bedingungen, dass der Grad der Umwandlung des Tetrachlorethylens in Trichloracetylchlorid höchstens gleich 80 % ist, was eine Reaktionsmischung ergibt, die im Wesentlichen das Trichloracetylchlorid und das überschüssige Tetrachlorethylen umfasst,
- einen Schritt der Teilfluorierung der erhaltenen Mischung durch Umsetzung der Mischung mit Fluorwasserstoffsäure, was den Erhalt einer Mischung von Trichloracetylfluorid und dem überschüssigen Tetrachlorethylen ermöglicht,
- einen Schritt der Trennung des Trichloracetylfluorids von dem überschüssigen Tetrachlorethylen,
- einen zweiten Schritt der Fluorierung des erhaltenen Trichloracetylfluorids in Trifluoracetylfluorid durch Umsetzung des Trichloracetylfluorids mit Fluorwasserstoff in der Gasphase in Gegenwart eines Katalysators auf Basis von Chrom,
- einen Schritt der Hydrolyse des erhaltenen Trifluoracetylfluorids.

24. Verfahren nach einem der Ansprüche 21 und 23, **dadurch gekennzeichnet, dass** man die erhaltene Trifluoressigsäure mit einer Base, vorzugsweise Natriumhydroxid oder Kaliumhydroxid, umsetzt.

## Claims

1. Process for the preparation of a haloacetyl fluoride, where the haloacetyl is chosen from chloroacetyls and bromoacetyls, **characterized in that** it comprises:
- a stage of preparation of a chloride or a bromide of said haloacetyl by photooxidation of a chlorinated or brominated ethylenic compound, said chlorinated or brominated ethylenic compound corresponding to the following formula: 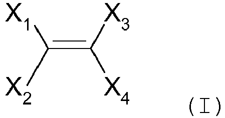 in said formula:
- X₁, X₂ and X₃ represent a hydrogen atom or a chlorine or bromine atom,
- X₄ represents a chlorine or bromine atom,
under conditions such that the degree of conversion of said ethylenic compound to said haloacetyl chloride or bromide is at most equal to 80%, resulting in a reaction mixture comprising essentially said haloacetyl chloride or bromide and said excess ethylenic compound,
- a stage of partial fluorination of the mixture obtained by reaction of the mixture with hydrofluoric acid, making it possible to obtain a mixture of fluoride of said haloacetyl and said excess ethylenic compound,
- a stage of separation of said haloacetyl fluoride and said excess ethylenic compound.

2. Process for the preparation of a fluoroacetyl fluoride, **characterized in that** it comprises:
- a stage of preparation of a haloacetyl fluoride according to the process of Claim 1;
- a second stage of fluorination of said haloacetyl fluoride obtained to give fluoroacetyl fluoride by reaction of said haloacetyl fluoride and hydrofluoric acid, in the gas phase, in the presence of a chromium-based catalyst.

3. Process for the preparation of a fluoroacetic acid, **characterized in that** it comprises:
- a stage of preparation of a fluoroacetyl fluoride according to the process of Claim 2;
- a stage of hydrolysis of the fluoroacetyl fluoride obtained, resulting in a fluoroacetic acid.

4. Process according to one of Claims 1 to 3, **characterized in that** said ethylenic compound is vinylidene chloride, trichloroethylene or tetrachloroethylene.

5. Process according to one of Claims 1 to 4, **characterized in that** the degree of conversion of said ethylenic compound to said haloacetyl chloride or bromide is between 30 and 80% and preferably between 60 and 75%.

6. Process according to one of Claims 1 to 5, **characterized in that** the mixture obtained comprises from 20 to 70% by weight of said haloacetyl chloride or bromide and from 70 to 20% by weight of said ethylenic compound and less than 10% of impurities.

7. Process according to one of Claims 1 to 6, **characterized in that** the partial fluorination of the mixture obtained is carried out by reaction of the mixture with liquid hydrofluoric acid, the ratio of the number of moles of hydrofluoric acid to the number of moles of said haloacetyl chloride or bromide being chosen between 1 and 10 and preferably between 1 and 5.

8. Process according to Claim 7, **characterized in that** the fluorination reaction is carried out at a temperature chosen between 80°C and 150°C and preferably between 100°C and 120°C, under autogenous pressure of the reactants.

9. Process according to either of Claims 7 and 8, **characterized in that**, at the end of the reaction, a two-phase mixture is obtained, the organic phase of which comprises from 50 to 80% by weight, preferably from 60 to 70% by weight, of said haloacetyl fluoride and from 50 to 20% by weight, preferably from 40 to 30% by weight, of said ethylenic compound.

10. Process according to one of Claims 1 to 9, **characterized in that** a distillation operation is carried out which makes it possible to separate said haloacetyl fluoride and said ethylenic compound and **in that** said ethylenic compound is optionally recycled.

11. Process according to one of Claims 2 to 10, **characterized in that** the fluorination of said haloacetyl fluoride is carried out by reaction of the latter with hydrofluoric acid in the presence of a fluorination catalyst: the ratio of the number of moles of hydrofluoric acid to the number of moles of said haloacetyl fluoride varies between 1 and 20 and preferably between 6 and 8.

12. Process according to Claim 11, **characterized in that** the fluorination catalyst comprises the oxides, halides, oxyhalides or inorganic salts of chromium optionally doped with a metal element, preferably nickel, cobalt, magnesium and zinc.

13. Process according to Claim 12, **characterized in that** the fluorination catalyst comprises an oxide of chromium, a fluoride of chromium or an oxyfluoride of chromium or else chromium doped with a metal element, preferably nickel or magnesium.

14. Process according to one of Claims 11 to 13, **characterized in that** the catalyst is subjected to activation by heat treatment and/or a fluorination treatment, it being possible for the activation to take place during the fluorination.

15. Process according to one of Claims 11 to 14, **characterized in that** the catalyst is a chromium(III) oxide.

16. Process according to Claim 11, **characterized in that** the temperature of the reaction is between 250°C and 400°C and preferably between 250°C and 300°C.

17. Process according to one of Claims 11 to 16, **characterized in that** the mixing of said haloacetyl fluoride and the hydrofluoric acid is carried out in a mixing region and then the mixture obtained is sent to the catalytic bed.

18. Process according to one of Claims 11 to 17, **characterized in that** the contact time, defined as the ratio of the apparent volume of catalyst to the flow rate of the gas stream, varies between 0.2 and 100 seconds, preferably between 5 and 50 seconds.

19. Process according to one of Claims 11 to 18, **characterized in that** the weight of substrate employed per weight of catalyst and per hour varies between 0.01 h⁻¹ and 2 h⁻¹ and preferably between 0.05 h⁻¹ and 0.5 h⁻¹.

20. Process according to one of Claims 11 to 19, **characterized in that**, at the end of the reaction, a gas phase is recovered which comprises the fluoroacetyl fluoride, the excess hydrofluoric acid and the hydrohalic acid formed by the reaction.

21. Process according to one of Claims 3 to 20, **characterized in that** a stage of hydrolysis of the fluoroacetyl fluoride to give fluoroacetic acid is subsequently carried out by bringing the gas stream into contact with water.

22. Process according to Claim 20, **characterized in that** the fluoroacetyl fluoride is recovered from the said gas stream comprising the flouoroacetyl fluoride, the hydrofluoric acid and the hydrohalic acid by condensing the said gas stream by lowering its temperature to between -40°C and 10°C, preferably between -20°C and 0°C, and by then distilling the condensed stream.

23. Process for the preparation of trifluoroacetic acid according to one of Claims 1 to 22, **characterized in that** it comprises:
- a stage of preparation of trichloroacetyl chloride by photooxidation of tetrachloroethylene under conditions such that the degree of conversion of the tetrachloroethylene to give trichloroacetyl chloride is at most equal to 80%, resulting in a reaction mixture essentially comprising the trichloroacetyl chloride and the excess tetrachloroethylene,
- a stage of partial fluorination of the mixture obtained by reaction of the mixture with hydrofluoric acid, making it possible to obtain a mixture of trichloroacetyl fluoride and the excess tetrachloroethylene,
- a stage of separation of the trichloroacetyl fluoride from the excess tetrachloroethylene,
- a second stage of fluorination of the trichloroacetyl fluoride obtained to give trifluoroacetyl fluoride by reaction of the trichloroacetyl fluoride and hydrofluoric acid, in the gas phase, in the presence of a chromium-based catalyst,
- a stage of hydrolysis of the trifluoroacetyl fluoride obtained.

24. Process according to either of Claims 21 and 23, **characterized in that** the fluoroacetic acid obtained is reacted with a base, preferably sodium hydroxide or potassium hydroxide.
